# EUROPEAN PATENT SPECIFICATION

(11) **EP 0 707 851 B1**
(45) Date of publication and mention of the grant of the patent: **09.01.2002**
(21) Application number: 95116380.7
(22) Date of filing: 14.02.1992
(51) Int. Cl.: A61K 31/35, C07D 311/32

(54) **Benzopyran phenol derivatives for the use as antibacterial, antiviral or wound healing agents**
Benzopyranphenolderivate zur Anwendung als bakterienhemmende, antivirale und wundheilende Mittel
Dérivés benzopyrannephénoliques pour l'utilisation comme agents bacteriostatiques, antiviraux et soignant les blessures

(30) Priority: 15.02.1991 US 656801; 17.12.1991 US 809420
(43) Date of publication of application: 24.04.1996
(62) Divisional of application: 92905459.1
(73) Proprietor: Fockerman, Jasmine, S-260 70 Ljungbyhed (SE); Fockerman, Michel, S-114 54 Stockholm (SE)
(72) Inventor: Marconius, Maria, S-121 74 Johanneshov (SE)
(74) Representative: Fagerlin, Heléne

(56) References cited:
- EP-A- 0 310 757
- US-A- 4 238 483
- US-A- 4 297 348
- GREENAWAY W ET AL: "IDENTIFICATION BY GAS CHROMATOGRAPHY-MASS SPECTROMETRY OF 150 COMPOUNDS IN PROPOLIS" ZEITSCHRIFT FUER NATURFORSCHUNG. SECTION C. BIOSCIENCES, vol. 46, no. 1/02, 1991, pages 111-121, XP000672605

## Description

The present invention concerns use of a composition comprising certain benzopyran phenol derivates for preparing antibacterial, antiviral, or wound healing agents.

The benzopyran phenol derivates according to the invention can be extracted from propolis. The ethanol extract of propolis can be used as prophylaxis for and against inflammations caused by certain viral infections (influenza, herpes) and is used as an antiinflammatory agent.

Propolis, also known as bee glue, is a natural product of bees. Bees collect propolis on the buds and other parts of plants, using it in their habitat to block up holes and cracks and also to isolate foreign bodies (insects and other living creatures) in the hive, thus preventing the spread of infections. It is also used by the bees to coat the cells of the honeycomb before storing their products such as honey and pollen in the honeycomb cells.

Propolis is a specific complex bioactive substance consisting of more than 60 compounds. The basic components are different resins, waxes, ethereal oils and pollen. In addition, major bioactive ingredients of propolis are vegetable dyes, of which the most important are the flavones or yellow dyes such as chrysin or tectochrysin, flavonones such as pinostrombin, pinocembrin and quercetin and their derivatives, and also flavonols such as rhamnocitrin, galangin and isoalpinin. It also contains aroma substances such as isovanillin and acetoxybetulinol, and aromatic acids such as cinnamic acid, benzoic acid, caffeic acid, ferulic acid and protocatechuic acid with their esters with benzyl alcohol, pentanol, phenylethyl alcohol and cinnamyl alcohol (E M Schneidewind, A Brige, H Kala, J Metzner and A Zsunke, in Die Pharmazie No. 34, 1979, 103).

USP 4.297,348 describes compositions for the treatment of acne comprising naringenin and naringin. The present composition however also comprises other compounds than naningenin.

It has now turned out that a composition comprising the compounds with the general formula I 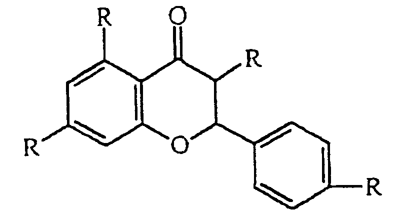 in which R is the same or different and represents H, OH or 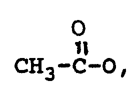 are components in propolis that can be used as antibacterial, antiviral or wound healing agents, which agents have better effects than propolis.

Such a composition may comprise Pinocembrin, 4H-1-benzopyran-4-one, 2,3-dihydro-5,7-dihydroxy-2-phenyl, Pinobanksin-3-acetate, 4H-1-benzopyran-4-one, 2,3-dihydro-5,7-dihydroxy-2-phenyl-3-acetate and Naringenin, 5,7,4'-trihydroxy-2,3-dihydro-2-phenyl-1,4-benzopyron or 5,7,4'-flavanone for use as an antibacterial, antiviral or wound healing agent.

The composition according to the invention is obtained by a process for preparing a mixture of the substances with formula I. According to this process a propolis product is extracted with a water solution containing 0,1 - 17 weight % NaCl. As antibacterial agent the extracted product may contain 0.5 - 2 weight % salt, preferably 0.9 % such as 0.9 % NaCl.

According to the present invention the propolis product is an organic solvent such as an alcoholic solution containing propolis. This alcoholic solution is added to a water solution containing 0.1-17 weight % of NaCl with a temperature of 30-95°C, and the mixture is kept at 30-95°C for 10 to 100 hours, whereafter the solution is freed from the bottom sediment.

The alcohol may be an alcohol containing 1-20 carbon atoms, preferably 1-5 carbon atoms, such as methanol, ethanol, propanol, butanol, especially ethanol. Preferably there is used an ethanolic propolis solution prepared according to German patent 88109824.8.

According to EP0 310 757, an ethanolic propolis product can be prepared by extracting propolis in a closed system at a low temperature, 0-20°C with an ethanol/water mixture at a volume ratio of 87 : 13 under ultrasonic treatment from 18 to 25 kHz for a short period such as 25 minutes, the resultant suspension being decanted and the clear propolis extract freed from the solid particles. Preferably, the propolis is ground to a particle size of 3 mm diameter before extraction. The propolis is ground in a suitable mill into fine particles (max. diameter 5 mm).

The alcoholic solution is preferably added to the salt solution in an amount of 1-60 % by volume counted on the sum of the volumes of the two solutions.

The propolis solution is preferably added to the salt solution drop by drop over about 1-7 hours, preferably 5 hours. A brown sediment is formed. The mixture is kept at 30-95°C for another 5-100 hours, preferably about 40-80 hours. A temperature of 50-70°C is preferred, especially 60°C. A light yellow solution is formed over a hard dark brown sediment. The propolis solution being alcoholic is evaporated during the process and the yellow extract that is obtained has about the same volume as the NaCl solution used.

The substances and the compositions according to the invention can be used as antimicrobial agents for human or veterinary use. They can be used for prophylaxis or treatment of inflammations or infections caused by gram positive or negative bacterias, viruses and fungi. The substances or mixtures thereof can be used as fodder, food-stuffs, hygienic articles, medicines and nature medicines. They can also be used as an antiseptic and analgesic and to improve the healing process of wounds. Tests discussed below also show that the extract according to the invention can be used effectively against bacteria causing gastric ulcer and vaginal infections.

The extract can also be used for preparing containers and tools for fodder and food-stuffs and for treatment of surgical instruments, dental instruments, cosmetic tools such as syringes, bandages, plasters, dressings, compresses and rinsing solutions. Further they can be used for treating all sorts of space such as operating rooms, rooms where animals are kept, places for preparing or storing foods and fodder.

When using the extraction process according to the invention there is obtained a product containing Pinocembrin, Pinobanksin-3-acetate, and Naringenin. The extract can be adjusted to contain preferably a physiological solution of 0.6-0.9, preferably 0.9 % NaCl. This is very suitable for treating or preventing bacterial infections such as mastitis i.e. inflammation in the udder. The saline extract could be prepared under sterile conditions and be injected directly in the udder. "Provet" ^{R} which is used today contains crude propolis (from Apipharm Co. Ltd.), Lanacolum, alcohol-cetyle-stearate, polyoxyethylene-sorbitan-monolaurate, vaseline and paraffin. This product "Provet" ^{R} must be introduced in the milk canal of each teat of the diseased mammal at the end of milking. "Provet" ^{R} thus consists of four disposable syringes for injection in each of the teats. Thus the composition according to the invention is easier to administer than the prior products.

In mice infected with Coxsackievirus B3 (CB3) the lifetime was significantly prolonged for mice treated prophylactically with the composition.

When tested as 1-10 % nose spray in a total of 58 patients no local or systemic allergic reactions were observed.

The substances and the compositions according to the invention can be used in any pharmaceutical form such as tablets, capsules, solutions for injections, solutions or spray for nasal treatment.

The administration forms may contain pharmaceutically acceptable carriers such as one or more compatible solid filler diluents or solid or liquid substances added to aid in the production of the pharmaceutical forms, such as lubricants to reduce friction and glidants to improve flow of the particulate mixtures. By "compatible" as used herein, is meant that the components are capable of being comingled without interacting in a manner which would substantially decrease the pharmaceutical efficacy.

Some examples of substances which can serve as pharmaceutical carriers are sugars, such as lactose, glucose and sucrose; starches, such as corn starch and potato starch; cellulose and its derivatives, such as sodium carboxymethylcellulose, ethylcellulose, cellulose acetate; powdered tragacanth; malt; gelatin; talc; stearic acid; magnesium stearate; zinc stearate; calcium sulphate; silicon dioxide; vegetable oils, such as peanut oil, cottonseed oil, sesame oil, olive oil, corn oil and oil of theobroma; polyols, such as propylene glycol, glycerine, sorbitol, mannitol, and polyethylene glycol; agar; and alginic acid; as well as other non-toxic compatible substances used in pharmaceutical formulations. Wetting agents such as sodium laural sulphate, as well as coloring agents, lubricants, excipients, stabilizers, antioxidants, and preservatives, can also be present.

For nasal treatment there can be used a physiological NaCl-solution containing 0.9 % by weight NaCl and 1-20 % propolis extract. For treatment of mastitis there can be used a 0.9 % NaCl-solution containing 20-50 % propolis extract, preferably 30 %.

The features and advantages of the present invention will be more clearly understood by reference to the following examples.

### Example 1 (Preparation of the starting material)

In the extraction vessel, 500 litres of an ethanol solution not less than 87 % by weight in water is prepared. While the mixture is being stirred, 20 kg of the ground propolis is added to the contents of the extractor. The batch is subjected to ultrasonic extraction (18 - 25 kHz) for 25 minutes, then left to stand for 2 hours, decanted and filtered.

The filtration is effected via a pressure filter with rapid filter-paper inserts. The clear propolis extract obtained is reduced to the required concentration of 5 to 80 % of the dry substance in a column concentrator in a 150 - 50 mm H₂O vacuum by means of a heat pump. The concentration is performed at a temperature of max. 20°C.

### Example 2

The extract obtained in example 1 was diluted to contain a dry substance weight of 10 % in ethanol. NaCl was dissolved in distilled water to a concentration of 0.9 %. 700 ml of the solution was placed in a beaker which was placed into a water bath. The temperature in the beaker was kept at 30°C. 300 ml of the propolis extract containing 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the level. By then a light yellow solution was formed the volume of which was 700 ml. The rest of the solution had evaporated during the heating. The beaker was left in the water bath until the content had reached room temperature. The precipitate was freed from the solution. Analysis by mass spectra of the yellow solution shows that it contains Pinocembrin and Pinobanksin-3-acetate.

### Example 3

The extract obtained in Example 1 was diluted to contain a dry substance weight of 10 % in ethanol. NaCl was dissolved in distilled water to a concentration of 10 %. 700 ml of the solution was placed in a beaker which was placed into a water bath. The temperature in the beaker was kept at 30°C. 300 ml of the propolis extract containing 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the water level constant. A light yellow solution having a volume of about 700 ml was formed. The rest of the solution had evaporated during the heating. The beaker containing the solution was left in the water bath until the content had reached room temperature. The precipitate was freed from the solution.

### Example 4

The extract obtained in example 1 was diluted to contain a dry substance weight of 10 % in ethanol. NaCl was dissolved in distilled water to a concentration of 15 %. 700 ml of the solution was placed in a beaker which was placed into a water bath. The temperature in the beaker was kept at 30°C. 300 ml of the propolis extract containing 10 weight % propolis in ethanol was added drop by drop to the NaCl solution during about 5 hours. A muddy brown precipitate was obtained. The temperature of the solution was controlled by keeping the water bath boiling and by continuously adding water during 15 hours to keep the level. A light yellow solution having the volume of about 700 ml was formed. The rest of the solution had evaporated during the heating. The beaker containing the solution was left in the water bath until the content had reached room temperature. The precipitate was freed from the solution.

### Example 5

The procedure of example 2 was repeated but with 800 ml of the NaCl solution and 200 ml of the propolis extract. The resultant solutions were analyzed by mass spectroscopy as follows. Two ml of the yellow extract were extracted with 2 ml ethyl acetate. Volumes of 1 and 5 microliters of the ethyl acetate phase were injected into a gas chromatograph coupled to a Jeol 300 mass spectrometer. The gas chromatograph comprised a 15 m long capillary column with unpolar stationary phase. Splitless injection was made 1 minute 240°, temperature processing; 45°, 1 minute, 10° per minute. The mass spectrometry analysis parameters were EI 70 EV 1.2 seconds/scan from mass 35 to 300. The inner standard was added to the sample with 430 µl of 0.7 µg/ml of Pinocembrin-7-methyl ether before the extraction was performed.

The inner standard Pinocembrin-7-methyl ether was also present in the sample, which makes analysis difficult. In order to be able to use the inner standard, the sample was chromatographed with and without inner standard so that the interference by the inner standard may be subtracted. Pinocembrin and pinobanksin-3-acetate were identified with reference spectra.

These results show that 2 ml of the extract, which is a 0.9 % NaCl solution, contains 200 ng/ml (20 x 10⁻⁸ g/ml) of Pinocembrin and 90 ng/ml (90 x 10⁹ g/ml) of Pinobanksin-3-acetate. There are no other detectable components in the extract.

### Example 6

The procedure of example 2 was repeated but there was used instead 770 ml of the NaCl solution and 230 ml of the propolis extract.

### Example 7

The procedure of example 2 was repeated but there was used instead 500 ml of the NaCl solution and 500 ml of the propolis extract.

The light yellow solution was further purified by dialysis against water. 50 ml of the extract was dialysed against 250 ml of distilled water through a tube (Model mw cataf 3500 Kebo AB Sweden).

Both the undialysed and the dialysed solution were tested by mass spectra with Solid Probe/µs. 1 µl of a mixture of the test solution and 96 % p.a. EtOH 1:1 was evaporated at about 60°C, chilled to 25°C and then heated 10°C/s to 375°C which temperature was maintained for about 4 min. Mass spectra from m/2 41-641 with cycle 1s was taken during this period. The mass spectra of the dialysed product are shown in figure 1 of which fig. 1a shows the temperature ramp, the four regions A-D and the total ion stream and fig. 1b shows the total ion stream after a numerical filter. Figure 1c shows the spectrum in the region A, figure 1d the spectrum of region B, figure 1e the spectrum of region C and 1f the spectrum of region D respectively. Figures 2a - 2f show the same spectra for the undialysed product. The mass spectra are interpreted to show that the product contains Pinocembrin and Pinobanksin-3-acetate.

### Example 8

The procedure of Example 7 was repeated but after adding the propolis exract drop by drop to the NaCl solution, the mixture was heated at 60°C for 76 hours. The yellow extract was separated from the precipitate and analyzed. Another dialysis equipment than in example 7 was used. Analysis of the yellow solution with mass spectra shows that it contains pinocembrin, pinobanksin-3-acetate and naringenin.

### Example 9

The procedure of Example 2 was repeated but after adding the propolis extract drop by drop to the NaCl solution, the mixture was heated at 60° for 78 hours. Another dialysis equipment than in example 7 was used. Analysis of the yellow solution with mass spectra shows that it contains pinocembrin, pinobanksin-3-acetate and naringenin.

### Example 10

The starting material was prepared as described in Example 1, and the clear propolis extract was reduced to a concentration of 20 weight %. Thereafter, the procedure of Example 7 was repeated, but after adding the propolis extract drop by drop to the NaCl solution, the mixture was heated at 60° for 72 to 78 hours. Another dialysis equipment than in example 7 was used. Analysis of the yellow solution with mass spectra shows that it contains pinocembrin, pinobanksin-3-acetate and naringenin. This extract is suitable as an antiviral agent, for example, through stimulation of the immune system (see Example 15).

The products of Examples 8, 9 and 10 have been analyzed with gas chromatography-masspectrometry.

### ANALYSIS PROCEDURE

Internal Standard (IS): 3.2 x 10⁻⁵ g/ml of Pinocembrin-7-methylether.
1: 1 ml sample was extracted with 1 ml ethylacetate.
2: 1 ml sample with 0.1 ml Internal Standard added was extracted with 1 ml ethylacetate.
3: 2 µl of every ethylacetate phase was injected on a Gas Chromatograph-Masspectrometer (GC-MS).

### GC PROGRAM

Column: 15 m x 0.25 mm with unpolar stationary phase.
Injection: 1 min splitless, 280°C.
Temperature program: 60°C (1 min) to 280°C with 5°C/min

### MASSPECTROMETER PARAMETERS

Scan: m/z 30-500 in 1.2 sec.
Ionization: Electron Impact (EI) 70 eV, Ionizationcurrent 50 µA

### RESULTS

Pinocembrin and Pinobanksin-3-acetate has been identified witn help of the reference spectra.

The analysis of Example 9a, Example 9b, Example 8 and Example 10 gave the following results concerning the amount of Pinocembrin and Pinobanksin-3-ac. and Naringenin.

| | Conc.Pinocembrin (milligram/liter) | Conc.Pinobanksin-3-ac. (milligram/liter) | Naringenin (mg/ml) |
|---|---|---|---|
| Ex.8 | 1.0 | 5.3 | 52 |
| Ex.9a | 5.3 | 5.9 | 45 |
| Ex.9b | 5.6 | 7.8 | 58 |
| Ex.10 | 10.0 | 13.0 | 59 |

Comment: The concentration of Pinocembrin and Pinobanksin-3-acetate given here should be taken as a hint of the amount of these compounds in the samples. There could be big errors in these concentrations because of, for example, differences in the recovery of the extraction between Pinocembrin, Pinobanksin-3-acetate, naringenin and Pinocembrin-7-methylether (Standard) and differences in the response on the masspectrometer between the compounds and the standard. To be able to do a more precise analysis of the amount of Pinocembrin and Pinobanksin-3-acetate one must use these compounds in pure form as standards.

### Example 11

Example 10 was repeated but there was used 500 ml water and 500 ml of 20 weight % propolis extract. The temperature was kept at 60°C and the extraction time ws 32 hours.

### Example 12

### Test of the bactericidal effects of the extracts according to examples 2 and 5.

The following strains were tested: Klebsiella oxytoca (Kl), Pseudomonas aeruginosa (P.a), a coagulase-negative staphylococci (SK-), Streptococcus uberis (Sru), Streptococcus agalactiae (Sra), Proteus mirabilis (P.m), Actinomyces pyogenes (A.p), Saccharomyces cerevisiae (S.ce), Candida pseudotropicalis (C.ps), (Kluveromyces marxianus).

The four propolis solutions were negative at sterility control tests made before the growth tests. All strains were tested once. They were cultivated in bovine broth at 37°C during 20 hours whereafter the broths contained 10⁶-10⁹ microorganisms per ml (see table I).

Samples of 0.1 ml were taken from each broth (dilution 10² or 10³) and added to 0.9 ml of the extract according to example 2 or 5 so as to make each mixture contain 10²-10⁵ bacteria/ml (see table II). The solutions were then incubated at 38°C. After 0 and 3 hours the bacteria were counted. The results are given in tables III-IV. After cultivation 3 hours there were no bacteria in the culture, which indicates that every bacterial group tested was dead after incubation three hours in the tested extracts according to Examples 2 and 5.

**Table I**

| Number of bacteria in the broths | | |
|---|---|---|
| Test 1 | | Test 2 |
| Ex 2 | | Ex 5 |
| Kl | 1.5∗10⁹ | 2.0∗10⁹ |
| P.a | 4.0∗10⁹ | 12∗10⁹ |
| SK- | 5.5∗10⁹ | 5.0∗10⁹ |
| Sru | 9.5∗10⁸ | 3.5∗10⁸ |
| Sra | 4.0∗10⁷ | 9.5∗10⁷ |
| P.m | 2.5∗10⁹ | 2.0∗10⁹ |
| A.p | 8.5∗10⁹ | 1.0∗10⁹ |
| S.ce | 1.5∗10⁷ | 3.0∗10⁶ |
| C.ps | 3.5∗10⁷ | 1.7∗10⁷ |

**Table II**

| Bacteria/ml extract | | |
|---|---|---|
| Ex 2 | | Ex 5 |
| Kl | 1.5∗10⁵ | 2.0∗105 |
| Pa | 4.0∗10⁵ | 12∗10⁵ |
| SK- | 5.5∗10⁵ | 5.0∗10⁵ |
| Sru | 9.5∗10⁵ | 3.5∗10⁵ |
| Sra | 4∗10⁴ | 9.5∗10⁴ |
| P.m | 2.5∗10⁵ | 2.0∗10⁵ |
| A.p | 8.5∗10⁵ | 1.0∗10⁵ |
| S.ce | 1.5∗10³ | 3.0∗10² |
| C.ps | 3.5∗10³ | 1.7∗10³ |

**Table III**

| time 0h | | | |
|---|---|---|---|
| Ex 2 | | Ex 5 | Ex 5 |
| Kl | 2.5∗10³ | 7.5∗10⁴ | 5.5∗10⁴ |
| P.a | 0 | 0 | 0 |
| SK- | 1∗10³ | 2.0∗10³ | 9.0∗10³ |
| Sru | 0 | 0 | 3.0∗10³ |
| Sra | 0 | 1.0∗10³ | 5.0∗10² |
| P.m | 2∗10⁵ | 9.5∗10⁴ | 5.5∗10⁴ |
| A.p | 0 | 0 | 1.0∗10⁵ |
| S.ce | 0 | 0 | 2.5∗10² |
| C.ps | 1∗10³ | 3.0∗10³ | 1.1∗10³ |

**Table IV**

| time 3h | | | | |
|---|---|---|---|---|
| Kl | 0 | 0 | 0 | 0 |
| P.a | 0 | 0 | 0 | 0 |
| SK- | 0 | 0 | 0 | 0 |
| Sru | 0 | 0 | 0 | 0 |
| Sra | 0 | 0 | 0 | 0 |
| P.m | 0 | 0 | 0 | 0 |
| A.p | 0 | 0 | 0 | 0 |
| S.ce | 0 | 0 | 0 | 0 |
| C.ps | 0 | 0 | 0 | 0 |

Control: 10 ml of the propolis bacterial suspension was added to 3 ml broth and cultivated at 37°C for 24 hours and transferred to a plate and cultivated 48 hours at 37°C. The growth was 0.

The following three strains were analyzed in another test: Staphylococcus aureus, Streptococcus dysgalactiae and Escherichia coli.

All propolis solutions were checked for existence of bacteria. They were all free from bacteria.

The bacteria were cultivated in broth for 24 hours. They then contained 10⁷-10⁸ bacteria per ml. From every broth culture 0.1 ml was taken out and mixed with 0.9 ml of propolis sulution and incubated at 37°C. Bacteria were counted after 0, 3, 24 and 48 hours.

Result: No bacteria could be found in the cultures after cultivating during 3 hours which indicates that all bacteria were dead.

### Example 13

The following three strains were analyzed in another test:
Staphylococcus aureus, Streptococcus dysgalactiae, and Escherichia coli.
All propolis solutions were checked for existence of viable bacteria, and were all found to be free from bacteria.

The bacteria are cultivated for 24 hours so that the bacterial solutions contain 10⁷-10⁸ bacteria per ml. From every broth culture, 0.1 ml is taken out and mixed with 0.9 ml propolis solution and incubated at 37°. Bacteria are counted after 0, 3, 24 and 48 hours. No bacteria are found in the cultures after cultivating during 3 hours which indicates that all bacteria were dead.

### Example 14

The following bacterial strains were used:
Helicobacter pylori NCTC 11736, International reference strain
Helicobacter pylori S-3, isolated in Orebro, Sweden
Helicobacter pylori S-6, isolated in Orebro, Sweden
Helicobacter pylori F-6, isolated in Helsinki, Finland
Helicobacter pylori 7-88, isolated in Helsinki, Finland
Campylobacter jejuni S-562, isolated in Orebro, Sweden
Campylobacter jejuni S-261, isolated in Orebro, Sweden
Staphylococcus epidermidis, laboratory stock strain
Stretpococcus agalactiae, group B, laboratory stock strain

Helicobacters and campylobacters are two important intestinal pathogenic bacteria that cause gastroenteritis. Helicobacter pylori is especially recognized as the cause of intestinal infections and the causal agent in peptic ulcer diseases. The Staphylococcus strain is a common hospital bacterial species, and Streptococcus agalactiae is a vaginal bacterium causing abortion. There is a great demand for an antibacterial therapy, without side effects, that could be used as a broad spectrum antibiotic on the above bacteria. Presently, the treatment comprises bismuth combined with two different antibiotics.

The strains used in the tests are cultured overnight in enriched Mueller Hinton Broth. Colony-forming units (CFU) are in the range of 1 x 10⁶/ml to 8 x 10⁷/ml for H. pylori and 2 x 10⁷/ml to 5 x 10⁸/ml for S. agalactiae and S. epidermidis, respectively.

A volume of 0.1 ml is taken from each broth culture and mixed with 0.9 ml of the resultant propolis solutions of examples 3 and 5, and are incubated at 37°C. From each of these mixtures after 0, 3, 24 and 48 hours, 0.1 ml is taken and cultured on blood agar medium.

No growth of bacteria occurs after 3 hours, which indicates that the tested bacteria are killed after 3 hours of incubation in the examined solutions. Each of these tests is repeated at least twice, with the same results.

### Example 15

### TOXICITY IN MICE

To obtain information on the effect of the product according to the invention as an adjuvant in animals the product of example 10 was tested in mice using a dose of 1 µg.

The animals were divided into two groups, each group consisting of six female 12 weeks old BALB/c mice.

The preparation was given to two groups of animals, either as subcutaneous (s/c) or intraperitonial (i/p) injections.

### RESULTS

No lethal effect was noticed during the observation period of three weeks. But minor local reactions were observed in two out of six animals inoculated s/c. The changes observed were total depletion of the hair at the site of injection.

### Example 16

IMMUNISATION OF MICE WITH Antigen mixed with the product of Example 10
Two groups (A and B) of 12 weeks old female BALB/c mice were used. Each group consisted of six animals.

The antigen (Ag) used for the immunisations was the envelope glycoproteins of Equine herpesvirus type-2 (EHV-2). The dose of antigen 5 µg protein for each of the two different preparations as measured by the Bradford method.

The groups of mice were immunised in the following manner:-
Group (A) received the Ag mixed with Freunds incomplete adjuvant (FIA).
Group (B) received the antigen mixed with 1 µg of the product of Example 10.

The mice were immunised twice four weeks apart. Blood samples were taken at week 4, 5 and 7 after the first immunisation. Serum was separated and inactivated for 1 hour at 56°C. Serum antibody responses to the envelope antigen were measured in ELISA.

**Table V**

| Serum antibody response of mice (mean values) immunised with 5 µg EHV-2 envelope antigen*; A) mixed with Freunds incomplete adjuvant; B) with 1 µg of Example 10. Results are given in dilutions that still give antibody response. | | | |
|---|---|---|---|
| Animal group | Weeks post immunisation | | |
| | 4 | 5 | 7 |
| A | 1/800 | 1/6400 | 1/6400 |
| B | 1/6400 | 1/102 400 | 1/102 400 |

| | | | |
|---|---|---|---|
| (*) The animals were immunised twice four weeks apart. | | | |

### RESULTS

Animals receiving antigen and the product of Example 10 in this test show about 16 times higher antibody titers than animals receiving antigen and FIA.

## Claims (Claims for the following Contracting State(s): AT, BE, CH, LI, DE, DK, FR, GB, IT, LU, MC, NL, SE)

1. Use of a composition comprising compounds according to the general formula I: 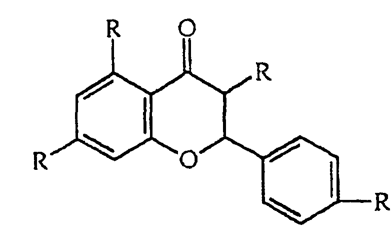 where R is the same or different and represents H-, OH- or CH₃-COO-, that is obtained by adding a propolis containing product such as an organic solvent containing propolis, preferably an alcoholic solution containing propolis, to a water solution containing 0.1-17 weight % of NaCl with a temperature of 30-95°C for 10-100 hours, whereafter the solution is freed from the bottom sediment for preparing a composition for use as an antibacterial, antiviral or wound healing factor.

2. Use according to claim 1 for preparing a composition for use as an antibacterial agent against Helicobacter pylori and/or Campylobacter jejuni infections.

3. Use according to claim 2 comprising pinobanksin-3-acetate, pinocembrin and naringenin, for preparing a composition for use as an antibacterial agent against Helicobacter pylori and/or Campylobacter jejuni infections.

## Claims (Claims for the following Contracting State(s): GR, ES)

1. A process for peparing a composition for use as an antibacterial, antiviral or a wound healing factor, **characterised in that** a composition comprising compounds according to the general formula I: 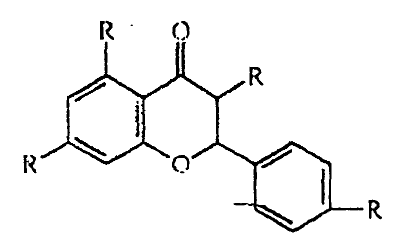 where R is the same or different and represents H-, OH- or CH₃-COO-, that is obtained by adding a propolis containing product such as an organic solvent containing propolis, preferably an alcoholic solution containing propolis, to a water solution containing 0.1-17 weight % of NaCl with a temperature of 30-95°C for 10-100 hours, whereafter the solution is freed from the bottom sediment.

2. A process according to claim 1 for preparing a composition for use as an antibacterial agent against Helicobacter pylori and/or Campylobacter jejuni infections.

3. A process according to claim 2, **characterised in** the use of pinobanksin-3-acetate, pinocembrin and naringenin, for preparing a composition for use as an antibacterial agent against Helicobacter pylori and/or Campylobacter jejuni infections.

## Patentansprüche (Patentansprüche für folgende(n) Vertragsstaat(en): AT, BE, CH, LI, DE, DK, FR, GB, IT, LU, MC, NL, SE)

1. Verwendung einer Zusammensetzung, umfassend Verbindungen gemäß der allgemeinen Formel (I): 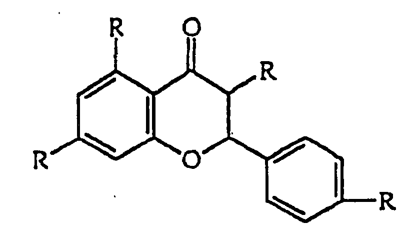 worin R gleich oder verschieden ist und H-, OH- oder CH₃-COO- darstellt, die durch Zugeben eines Propolis enthaltenden Produkts, wie ein Propolis enthaltendes organisches Lösungsmittel, bevorzugt eine Propolis enthaltende alkoholische Lösung, zu einer 0,1 bis 17 Gew.-% NaCl enthaltenden Wasserlösung mit einer Temperatur von 30 bis 95°C über 10 bis 100 h, wonach die Lösung von dem Bodensediment befreit wird, erhalten wird, zum Herstellen einer Zusammensetzung für die Verwendung als ein antibakterieller, antiviraler oder Wundheilungsfaktor.

2. Verwendung gemäß Anspruch 1 zum Herstellen einer Zusammensetzung für die Verwendung als ein antibakterielles Mittel gegen Helicobacter pylori- und/oder Campylobacter jejuni-Infektionen.

3. Verwendung gemäß Anspruch 2, umfassend Pinobanksin-3-acetat, Pinocembrin und Narigenin, zum Herstellen einer Zusammensetzung für die Verwendung als ein antibakterielles Mittel gegen Helicobacter pylori- und/oder Campylobacter jejuni-Infektionen.

## Patentansprüche (Patentansprüche für folgende(n) Vertragsstaat(en): GR, ES)

1. Verfahren zum Herstellen einer Zusammensetzung für die Verwendung als ein antibakterieller, antiviraler oder Wundheilungsfaktor, **gekennzeichnet dadurch, daß** eine Zusammensetzung, umfassend Verbindungen gemäß der allgemeinen Formel (I): 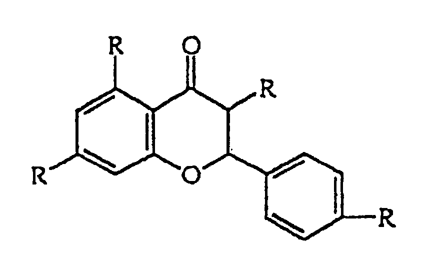 worin R gleich oder verschieden ist und H-, OH- oder CH₃-COO- darstellt, durch Zugeben eines Propolis enthaltenden Produkts, wie ein Propolis enthaltendes organisches Lösungsmittel, bevorzugt eine Propolis enthaltende alkoholische Lösung, zu einer 0,1 bis 17 Gew.-% NaCl enthaltenden Wasserlösung mit einer Temperatur von 30 bis 95°C über 10 bis 100 h, wonach die Lösung von dem Bodensediment befreit wird, erhalten wird.

2. Verfahren gemäß Anspruch 1 zum Herstellen einer Zusammensetzung für die Verwendung als ein antibakterielles Mittel gegen Helicobacter pylori- und/oder Campylobacter jejuni-Infektionen.

3. Verfahren gemäß Anspruch 2, **gekennzeichnet durch** die Verwendung von Pinobanksin-3-acetat, Pinocembrin und Narigenin, zum Herstellen einer Zusammensetzung für die Verwendung als ein antibakterielles Mittel gegen Helicobacter pylori- und/oder Campylobacter jejuni-Infektionen.

## Revendications (Revendications pour l'(les) Etat(s) contractant(s) suivant(s): AT, BE, CH, LI, DE, DK, FR, GB, IT, LU, MC, NL, SE)

1. Utilisation d'une composition comprenant des composés selon la formule générale I : 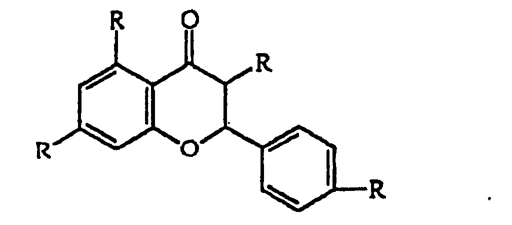 où les R sont identiques ou différents et représentent H-, OH- ou CH₃-COO-, que l'on obtient par addition d'un produit contenant du propolis, tel qu'un solvant organique contenant du propolis, de préférence une solution alcoolique contenant du propolis, à une solution aqueuse contenant 0,1 à 17 % en masse de NaCl à une température de 30-95°C pendant 10-100 heures, après quoi la solution est débarrassée du sédiment déposé pour préparer une composition utile en tant qu'agent antibactérien, agent antiviral ou facteur de cicatrisation des plaies.

2. Utilisation selon la revendication 1, pour la préparation d'une composition utile en tant qu'agent antibactérien contre des infections à *Helicobacter pylori* et/ou *Campylobacter jejuni*.

3. Utilisation selon la revendication 2, comprenant du 3-acétate de pinobanksine, de la pinocambrine et de la naringénine, pour la préparation d'une composition utile en tant qu'agent antibactérien contre des infections à *Helicobacter pylori* et/ou *Campylobacter jejuni*.

## Revendications (Revendications pour l'(les) Etat(s) contractant(s) suivant(s): GR, ES)

1. Procédé pour la préparation d'une composition utile en tant qu'agent antibactérien, agent antiviral ou facteur de cicatrisation des plaies, **caractérisé en ce qu'**une composition comprenant des composés selon la formule générale I : 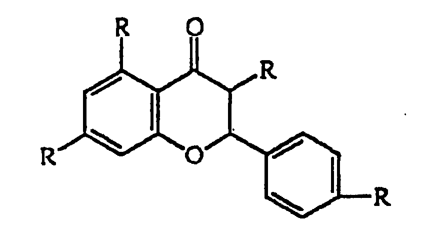 où les R sont identiques ou différents et représentent H-, OH- ou CH₃-COO-, est obtenue par addition d'un produit contenant du propolis, tel qu'un solvant organique contenant du propolis, de préférence une solution alcoolique contenant du propolis, à une solution aqueuse contenant 0,1 à 17 % en masse de NaCl à une température de 30-95°C pendant 10-100 heures, après quoi la solution est débarrassée du sédiment déposé.

2. Procédé selon la revendication 1, pour la préparation d'une composition utile en tant qu'agent antibactérien contre des infections à *Helicobacter pylori* et/ou *Campylobacter jejuni*.

3. Procédé selon la revendication 2, **caractérisé par** l'utilisation du 3-acétate de pinobanksine, de la pinocambrine et de la naringénine pour la préparation d'une composition utile en tant qu'agent antibactérien contre des infections à *Helicobacter pylori* et/ou *Campylobacter jejuni*.
